# EUROPÄISCHE PATENTSCHRIFT

(11) **EP 0 269 819 B1**
(45) Veröffentlichungstag und Bekanntmachung des Hinweises auf die Patenterteilung: **01.12.1993**
(21) Anmeldenummer: 87115236.9
(22) Anmeldetag: 17.10.1987
(51) Int. Cl.: C08G 18/10, C08G 18/28, C08G 18/73, C08G 18/75, C08G 18/83, A61K 6/10, C08G 18/48, C08G 18/12

(54) **Verwendung von Poly-(ether-urethan-harnstoff)Polyadditionsprodukten als Abformmassen im Dentalbereich**
Use of Poly(ether-urethane-urea) polyaddition products as dental impression materials
Utilisation de produits de polyaddition de polyéther-uréthane-urée comme masse d'empreinte dentaire

(30) Priorität: 30.10.1986 DE 3636974
(43) Veröffentlichungstag der Anmeldung: 08.06.1988
(73) Patentinhaber: BAYER AG, 51368 Leverkusen (DE)
(72) Erfinder: Müller, Hanns Peter, Dr., D-5060 Bergisch-Gladbach 2 (DE); Schwabe, Peter, Dr., D-5090 Leverkusen 1 (DE)

(56) Entgegenhaltungen:
- EP-A- 0 096 249
- EP-A- 0 158 893
- US-A- 3 632 557
- US-A- 4 374 237

## Beschreibung

Die vorliegende Erfindung betrifft die Verwendung von Abmischungen enthaltend Ether-, Urethan- und Harnstoffgruppen enthaltende Polyadditionsprodukte mit Alkoxysilylendgruppen, und einen Vernetzer zur Herstellung von dimensionsstabilen Abform- bzw. Dubliermassen, die im Dentalbereich Anwendung finden.

Die Polyadditionsprodukte können insbesondere in Form von Pasten zur Herstellung genauer Abformungen bezahnter, teilbezahnter und unbezahnter Kiefer sowie von Gipsmodellen eingesetzt werden. Die zu diesem Zweck eingesetzte Mischung besteht aus einem Mehrkomponentensystem, welches erhältlich ist durch Mischen des einen Vernetzer enthaltenden Polyadduktes mit einer Katalysatorkomponente und Wasser. Diese Mischung vernetzt bei Raumtemperatur oder Körpertemperatur schnell und bildet dabei den Abdruck.

Abformmassen, die im Dentalbereich Anwendung finden, sind an sich bekannt (vgl. z.B. R.G. Craig, Restorative Dental Materials, The C.V. Moosbe-Comp. St. Louis, Toronto, London, 1980, S. 1979 ff). An derartige Materialien werden insgesamt sehr hohe Anforderungen gestellt:
1. Angenehmer Geruch, Geschmack und ästhetisches Aussehen.
2. Die Massen dürfen keine toxischen oder irritierenden Bestandteile enthalten.
3. Die Massen müssen eine gute Lagerstabilität aufweisen.
4. Die Massen müssen wirtschaftlich herstellbar sein und präzise Abformungen ergeben.
5. Die Massen müssen leicht zu handhaben sein, ohne großen technischen Aufwand.
6. Die Härtungscharakteristik muß den klinischen Erfordernissen entsprechen.
7. Die ausgehärteten Massen müssen elastisch sein und dürfen sich nicht unter Zugbeanspruchung bleibend verformen.
8. Die ausgehärteten Massen müssen eine ausreichende Druckfestigkeit besitzen und dürfen nicht brechen.
9. Die ausgehärteten Massen müssen bei Raumtemperatur und normaler Luftfeuchtigkeit solange dimensionsstabil sein, daß im Labor in angemessener Zeit exakte Gipsabdrücke hergestellt werden können.
10. Die ausgehärteten Massen dürfen keine Gipsschädigungen hervorrufen und müssen mit anderen Abformmaterialien kompatibel sein.

Die bekannten Abformmassen des Standes der Technik erfüllen insgesamt nicht alle oben genannten Anforderungen. So weisen Alginat-Abformmassen durch schnelles Verdunsten des Wassers Schrumpf auf und verspröden.

Polysulfid-Abformmassen sind dunkel gefärbt und enthalten Blei- oder Kupferverbindungen als Beschleuniger.

Polyether-Abformmassen enthalten Ethyleniminvernetzer und Polysiloxan-Abformmassen ergeben auf Grund ihrer Hydrophobie gelegentlich fehlerhafte Abdrücke, bedingt durch Feuchtigkeit in der Mundhöhle.

Aus der US-A-3,632,557 sind vernetzbaresilikonendgestoppte organische Polymere auf der Basis von Isocyanatendgruppen bekannt geworden, deren Anwendung im humanmedizinischen Bereich aus toxikologischen Gründen und wegen einer zu langen Aushärtungszeit jedoch ausscheidet. Die EP-A-96249 betrifft hydroxylgruppenhaltige Polymermischungen, die jedoch als Haftkleber eingesetzt werden.

Die vorliegende Erfindung liegt daher die Aufgabe zugrunde, die Nachteile der bekannten Abformmassen zu vermeiden und darüber hinaus vernetzbare Polyadditionsprodukte zur Verfügung zu stellen, die neben einer physiologischen Unbedenklichkeit auch alle anderen aufgeführten Anforderungen erfüllen.

Die vorliegende Erfindung betrifft somit die Verwendung von Ether-, Urethan-, Harnstoffgruppen und Alkoxysilylendgruppen enthaltenden Polyadditionsprodukten mit einer überwiegend linearen Molekülstruktur, ausschließlich aliphatisch oder cycloaliphatisch gebundenen Ether-, Urethan- und Harnstoffsegmenten und einem mittleren Molekulargewicht Mn von 800 - 20 000,
gekennzeichnet durch
a) einen Gehalt an Polyethergruppen von 25 bis 90 Gew.-%, vorzugsweise von 50 bis 80 Gew.-% auf 100 Gew.-% des Polyadditionsprodukts,
b) einen Gehalt an Urethangruppen 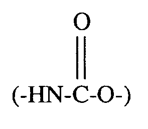 von 0,5 bis 10 Gew.-%, vorzugsweise von 1 bis 8 Gew.-% auf 100 Gew.-% des Polyadditionsprodukts,
c) einen Gehalt an Harnstoffgruppen 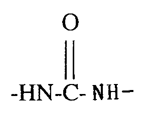 von 0,5 bis 10 Gew.-%, vorzugsweise 1 - 8 Gew.-% auf 100 Gew.-% des Polyadditionsprodukts und
d) einen Gehalt an endständigen Alkoxysilylgruppen 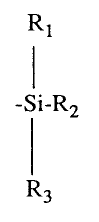 von 1 bis 25 Gew.-%, vorzugsweise 2 bis 10 Gew.-% auf 100 Gew.-% des Polyadditionsprodukts, wobei die Alkoxysilylgruppen im Polyaddukt Bestandteil von Strukturelementen folgender Formel sind:

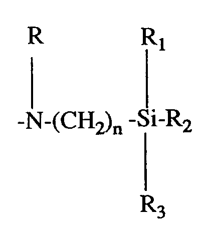

worin
- n: eine Zahl von 1 bis 6, bevorzugt die Zahl 3 darstellt,
- R: Wasserstoff oder -(CH₂)ₙ-SiR₁R₂R₃ bedeutet,
- R₁R₂R₃: C₁-C₄-Alkoxy, bevorzugt Methoxy oder Ethoxy bedeuten
in Abmischungen enthaltend als Vernetzer Wasser und organische und/oder anorganische Säuren in Gewichtsverhältnis von 1:0,01 bis 1:40 zur Herstellung von Abform-bzw. Dubliermassen in der Dentaltechnik.

Die erfindungsgemäß eingesetzten Polyadditionsprodukte können hergestellt werden, indem man aliphatische und/oder cycloaliphatische Diisocyanate mit Dihydroxypolyethern des mittleren Molgewichtsbereiches Mn von 300 bis 6000 umsetzt, wobei man gegebenenfalls zusätzlich noch aliphatische und/oder cycloaliphatische zweiwertige Alkohole eines mittleren Molgewichtes Mn von 62 bis < 300 zusetzt, und die erhaltenen Prepolymeren mit Alkoxysilylmonoaminen umsetzt, wobei man gegebenenfalls noch aliphatische und/oder cycloaliphatische Diamine mit primären Aminogruppen mit einem Molekulargewicht Mn von 60 bis 300 mitverwenden kann,
wobei man
a) Alkoxysilylmonoamine der Formel

   HRN-(CH₂)ₙ-SiR₁R₂R₃

   mit den oben näher bezeichneten Bedeutungen der Reste R,R₁,R₂,R₃, einsetzt, und
b) pro 1 Gew.-Tl. Dihydroxypolyether
   - 0,05 bis 1,5, bevorzugt 0,1 bis 0,5 Gew.-Tl. des Diisocyanates,
   - 0 bis 0,6, bevorzugt 0 bis 0,2 Gew.-Tl. des zweiwertigen Alkoholes,
   - 0,02 bis 0,40, bevorzugt 0,05 bis 0,2 Gew.-Tl. des Alkoxysilylmonoamins und
   - 0 bis 0,6, bevorzugt 0 bis 0,2 Gew.-Tl. des Diamins,
einsetzt.

Die Umsetzung der Komponenten erfolgt in der Regel bei Temperaturen von 20 bis 150°C, bevorzugt von 60 bis 120°C.

Die gegebenenfalls eingesetzten Diamine dienen zur Einstellung des jeweils gewünschten Molekulargewichtes.

Geeignete Diisocyanate sind insbesondere solche mit aliphatisch und/oder cycloaliphatisch gebundenen Isocyanatgruppen der Formel Q(NCO)₂, in welcher Q für einen aliphatischen Kohlenwasserstoffrest mit 2 bis 12 Koblenstoffatomen oder einen cycloaliphatischen bzw. gemischt aliphatisch-cycloaliphatischen Kohlenwasserstoffrest mit 4 bis 15 Kohlenstoffatomen steht.

Beispiele derartiger Diisocyanate sind Ethylendiisocyanat, Tetramethylendiisocyanat, Hexamethylendiisocyanat, Dodecamethylendiisocyanat, Cyclobutan-1,3-diisocyanat, Cyclohexan-1,3- und 1,4-diisocyanat oder 1-Isocyanato-3,3,5-trimethyl-5-isocyanatomethyl-cyclohexan bzw. beliebige Gemische derartiger Diisocyanate. Vorzugsweise werden beim erfindungsgemäßen Verfahren cycloaliphatische bzw. gemischt aliphatisch-cycloaliphatische Diisocyanate eingesetzt. Besonders bevorzugt ist 1-Isocyanato-3,3,5-trimethyl-5-isocyanato-methyl-cyclohexan (Isophorondiisocyanat).

Geeignete Dihydroxypolyether sind ebenfalls solche der an sich bekannten Art und werden z.B durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von BF₃, oder durch Anlagerung dieser Epoxide, gegebenenfalls im Gemisch oder nacheinander, an Startkomponenten mit reaktionsfähigen Wasserstoffatomen wie Alkohole oder Amine, z.B. Wasser, Ethylenglykol, Propylenglykol-(1,3) oder -(1,2), 4,4'-Dihydroxydiphenylpropan, Anilin, hergestellt. Vielfach sind solche Polyether bevorzugt, die überwiegend (bis zu 90 Gew.-%, bezogen auf alle vorhandenen OH-Gruppen im Polyether) primäre OH-Gruppen aufweisen.

Geeignete Diamine sind vorzugsweise primäre Aminogruppen aufweisende aliphatische, cycloaliphatische oder gemischt aliphatisch-cycloaliphatische Diamine des Molekulargewichtsbereichs 60 bis 300. Beispiele sind Ethylendiamin, Tetramethylendiamin, Hexamethylendiamin, 4,4'-Diamino-dicyclohexylmethan, 1,4-Diaminocyclohexan, 4,4'-Diamino-3,3'-dimethyl-dicyclohexylmethan oder 1-Amino-3,3,5-trimethyl-5-aminomethyl-cyclohexan (Isophorondiamin). Ganz besonders bevorzugt werden 4,4'-Diaminodicyclohexylmethan oder das zuletzt genannte Isophorondiamin eingesetzt.

Als zweiwertige Alkohole kommen z.B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol, 1,4-Bis-hydroxymethyl-cyclohexan, 2-Methyl-1,3-propandiol, 3-Methylpentandiol-(1,5), ferner Diethylenglykol, Triethylenglykol, Tetraethylenglykol, Polyethylenglykole, Dipropylenglykol, Polypropylenglykole, Dibutylenglykole und Polybutylenglykole in Frage.

Geeignete Alkoxysilylmonoamine sind ebenfalls bekannt, hierbei handelt es sich vorzugsweise um die technisch gut zugänglichen γ-Aminopropyl-tri-C₁-C₄-alkoxysilane bzw. um Bis-(3-C₁-C₄-alkoxysilylpropyl)-amine, ganz besonders bevorzugt ist γ-Aminopropyl-triethoxysilan.

Die Erfindung betrifft weiterhin eine Abmischung des geschilderten Polyadditionsproduktes mit einem Vernetzungsmittel, wobei als Vernetzungsmittel bevorzugt Tetrakieselsäureester, insbesondere Tetraethoxysilan, und Polyalkoxypolysiloxane verwendet werden.

Bezogen auf 1 Gewichtsteil des Polyadditionsproduktes können 0,01 bis 5, bevorzugt 0,1 bis 1 Gewichtsteile des Vernetzungsmittels eingesetzt werden.

Die Herstellung dieser Mischung erfolgt in einer dem Fachmann bekannten Weise, beispielsweise durch Mischen der Komponenten bei Raumtemperatur gegebenenfalls bei mäßig erhöhter Temperatur bis 60°C.

Für den Einsatz des erfindungsgemäßen Polyadditionsproduktes zu Dentalzwecken ist es empfehlenswert, der Abmischung bestehend aus Polyadditionsprodukt und Vernetzungsmittel noch einen Katalysator und Wasser zuzusetzen, damit die Vernetzung beschleunigt wird. Speziell Abformmassen, die in der Mundhöhle eines Patienten eingesetzt werden, sollten schnell dimensionsstabil aushärten.

Die Menge an Komponente B in den erfindungsgemäß zu Verwendenden Mischungen sollte bevorzugt 40 Gew.-% nicht überschreiten, wobei die gegebenenfalls mitbenutzten Füllstoffmengen unberücksichtigt bleiben.

Sowohl die Komponente A also auch die Komponente B können weitere übliche Hilfs- und/oder Zuschlagstoffe enthalten.

Unter Hilfs- und/oder Zuschlagsstoffen versteht man beispielsweise Paraffine, Emulgatoren, Glycerin, Sorbit, Füllstoffe, z.B. Quarz-Cristobalitmehle, Calciumsulfat, Diatomeenerde, Silikate, gefälltes und pyrogenhergestelltes Siliciumdioxid mit unbeladener oder beladener Oberfläche.

Farbstoffe können zur Unterscheidung der Komponenten A und B eingesetzt werden, damit keine Verwechselungen beim Abmischen auftreten.

Weiterhin ist es zweckmäßig, Indikatoren wie z.B. Bromphenolblau zur Mischkontrolle zu verwenden. Solche Indikatoren zeigen an, wann das Abmischen der Komponenten A und B beendet werden kann. Das Ende wird durch Farbumschlag angezeigt.

Unter den Protonenspendern versteht man organische und/oder anorganische Säuren wie saure lonenaustauscher, Phosphorsäure, Dibutylphosphorsäure, verd. Schwefelsäure, Weinsäure, Citronensäure oder Adipinsäure. Bevorzugt ist die Weinsaure.

Die gebrauchsfähige Abformmasse härtet dann - je nach Einstellung - innerhalb der bei der Anwendung üblichen Zeit - abhängig von der Umgebungstemperatur - zu einem jedes Detail genau wiedergebenden elastischen Material aus.

Von dem ausgehärteten Material lassen sich Gipsmodelle oder Abformungen mit handelsüblichen Abformmassen herstellen.

Es ist als äußerst überraschend anzusehen, daß die erfindungsgemäßen Formulierungen Abformmassen ergeben, die beim Wiederabformen z.B. mit Gips keine Gipsschädigungen hervorrufen und daß die ausgehärteten Massen, obwohl sie Wasser enthalten, über einen längeren Zeitraum dimensionsstabil bleiben als vergleichsweise Hydrokolloide.

Die erfindungsgemäß zu verwendenden Massen zeichnen sich weiterhin dadurch aus, daß sie metallfrei aushärten und das vernetzte Material von eventuell vorhandener Feuchtigkeit gut benetzt wird.

Beispielhaft sei folgende Zusammensetzung der Komponente A erwähnt:
a) 20-50 Gew.- % Polyadditionsprodukt,
b) 5-30 Gew.- % Tetraethoxysilan,
c) 0-15 Gew.- % Paraffine,
d) 0-70 Gew.- % Füllstoffe,
e) 0- 5 Gew.- % Emulgator,
f) 0,01 Gew.- % Indikator,
beispielhaft folgende der Komponente B:
g) 5-30 Gew.- % organische oder anorganische Säuren,
h) 0,1-10 Gew.- % Wasser,
i) 0-10 Gew.- % Emulgator,
k) 0-10 Gew.- % Glycerin,
l) 0-70 Gew.- % Füllstoffe,
m) 0-10 Gew.- % Paraffine.

Die folgenden Beispiele dienen der weiteren Erläuterung der Erfindung.

### Beispiele

### Beispiel 1

800 g (0,4 Mol OH) eines linearen Polyethers des M̅G 4000 (hergestellt durch Polyaddition von 87 Gew.-Tlen Propylenoxid auf Propylenglykol und anschließender Polyaddition von 13 Gew.-Tlen Ethylenoxid) werden 30 Minuten bei 120°C im Wasserstrahlvakuum entwässert. Danach werden dem Ansatz 88,8 g (0,8 Mol NCO) Isophorondiisocyanat (im folgenden IPDI genannt) zugefügt und das ganze 4 Std. bei 120 bis 140°C unter Stickstoff gerührt. Anschließend wird die NCO-Zahl des Prepolymeren bestimmt:
NCO gef.: 1,70 %
NCO ber.: 1,89 %.

Zu dem Prepolymeren werden dann 88,4 g (0,4 Mol) 3-Aminopropyltriethoxysilan unter Rühren und Stickstoff bei 30°C innerhalb von 30 Minuten zugetropft. Dabei erwärmt sich die Mischung auf 50°C. Man läßt anschließend bei 60°C 30 Min. nachreagieren. Im resultierenden Polyurethan-polyharnstoff läßt sich IR-spektroskopisch kein NCO mehr nachweisen. Man erhält auf diese Weise nach Abkühlen ein fast farbloses, klares, viskoses Produkt mit einem Gehalt an^{.}-HN-CO-NH- : 1,237 Gew.-% und einem Gehalt an endständigen Alkoxysilylgruppen : 6,22 Gew.-%.

### Beispiel 2

1000 g (0,5 Mol OH) eines linearen Polyethers des M̅G 4000 (hergestellt durch Polyaddition von 70 Gew.-Tlen Propylenoxid auf Propylenglykol und anschließender Polyaddition von 30 Gew.-Tlen Ethylenoxid) werden wie in Beispiel 1 entwässert. Bei 40°C werden dem Ansatz 111 g (1 Mol NCO) IPDI in einem Guß zugegeben und anschließend 1 Tropfen Zinnoktoat. Unter Rühren und Stickstoff wird der Ansatz auf 110°C geheizt und 40 Min. bei dieser Temperatur belassen. Anschließend wird die NCO-Zahl des Prepolymeren bestimmt:
NCO gef.: 1,69 %
NCO ber.: 1,89 %.

Nach Abkühlen auf 60°C tropft man unter Rühren und Stickstoff 110,5 g (0,5 Mol) 3-Aminopropyl-triethoxysilan innerhalb von 10 Min. ein. Dabei erwärmt sich der Ansatz auf 75°C. Man läßt den Ansatz ohne weitere Heizung 30 Min. nachreagieren. Im resultierenden Polyurethan-polyharnstoff ist IR-spektroskopisch kein freies NCO mehr nachweisbar. Der Polyurethan-polyharnstoff ist farblos, klar und gut gießfähig mit einem Gehalt an -NH-CO-NH- : 2,37 Gew.-% und einem Gehalt an endständigen Alkoxysilylgruppen : 6,22 Gew.-%.

### Beispiel 3

1000 g (1 Mol OH) eines linearen Polyethers des M̅G 2000 (hergestellt durch Polyaddition von gleichen Gew.-Tlen Propylenoxid und Ethylenoxid auf Propylenglykol) werden wie im Beispiel 1 entwässert. Bei 80°C werden dem Ansatz 166,5 g (1,5 Mol NCO) IPDI in einem Guß zugegeben und anschließend 1 Tropfen Zinnoktoat. Unter Stickstoff wird der Ansatz bei 120°C 4 Std. gerührt. Anschließend wird die NCO-Zahl des Prepolymeren bestimmt:
NCO gef.: 1,79 %.
NCO ber.: 1,80 %.

Nach Abkühlen auf 60°C tropft man unter Rühren und Stickstoff 110,5 g (0,5 Mol) 3-Aminopropyl-triethoxysilan zu. Danach ist der Ansatz NCO frei, Der Polyurethan-polyharnstoff ist eine farblose, klare, hochviskose Flüssigkeit mit einem Gehalt an -NH-CO-NH- : 2,27 Gew.-% und einem Gehalt an endständigen Alkoxysilylgruppen von 5,95 Gew.-%.

### Beispiel 4

Man verfährt wie im Beispiel 3 beschrieben, nur mit dem Unterschied, daß dem resultierenden Polyurethan-polyharnstoff nach der Herstellung 639 g Tetraethoxysilan zugegeben werden. Die homogene Mischung ist klar, farblos und besitzt eine Viskosität η_{25°C}: 2016 mPas.

Die gebrauchsfertige Mischung enthält 1,51 Gew.-% -NH-CO-NH- und 3,96 Gew.-% entständig gebundene Alkoxysilylgruppen und zusätzlich 50 Gew.-% Tetraethoxysilan.

### Beispiel 5

In einem Planetenmischwerk wird unter Verwendung des in Beispiel 3 beschriebenen Polyurethan-polyharnstoffs eine erfindungsgemäße Abformmasse folgendermaßen hergestellt:
- Komponente A: 30 Gew.-Tle. Polyurethan-polyharnstoff aus Beispiel 3
15 Gew.-Tle. Tetraethoxysilan
11 Gew.-Tle. Paraffin
41 Gew.-Tle. Füllstoff
2,99 Gew.-Tle. Emulgator
0,01 Gew.-Tle. Indikator
werden in der angegebenen Reihenfolge gemischt. Die Mischzeit bei 25°C beträgt 30 Min. bei 50 U/Min.
- Komponente B: 25 Gew.-Tle. 15 %ige Weinsäure
4 Gew.-Tle. Wasser
10 Gew.-Tle. Emulgator
3 Gew.-Tle. Glycerin
4 Gew.-Tle. Paraffin
54 Gew.-Tle. Füllstoffe
werden in der angegebenen Reihenfolge gemischt. Die Mischzeit bei 25°C beträgt 30 Min. bei 50 U/Min.

Gleiche Gewichtsteile der Komponenten A und B werden intensiv vermischt und vernetzen zu einem elastischen Material innerhalb von 3 Minuten.

Die physikalischen Werte des Materials, gemessen nach ISO 4823, sind wie folgt:

| | |
|---|---|
| Verarbeitungszeit | 2,5 Min. |
| Konsistenz | 45 mm |
| Bleibende Deformation | 2,3 %. |

Die Dimensionsänderung, gemessen nach ADA 19 bei Trockenlagerung, beträgt:

| | |
|---|---|
| nach 6 Minuten | 0,6 % |
| nach 10 Minuten | 1,1 % |
| nach 120 Minuten | 2,2 %. |

Die elastische Verformung, gemessen nach ADA 19, beträgt 9 %.

Zum Vergleich:
Die Dimensionsänderung einer Hydrokolloidabformmasse, kann bei Trockenlagerung nicht gemessen werden, da diese zu schnell austrocknen und unter Schrumpfung verspröden.

### Beispiel 6

In einem Planetenmischwerk wird unter Verwendung des in Beispiel 3 beschriebenen Polyurethan-polyharnstoffs eine erfindungsgemäße Abformmasse folgendermaßen hergestellt:
- Komponente A: 50 Gew.-Tle. Polyurethan-polyharnstoff aus Beispiel 2
2 Gew.-Tle. Aerosil (SiO₂)
43 Gew.-Tle. Füllstoff (Quarzmehl)
5 Gew.-Tle. Emulgator
0,05 Gew.-Tle. Indikator (Bromphenolblau)
werden in der angegebenen Reihenfolge gemischt. Die Mischzeit bei 25°C beträgt 30 Min. bei 50 U/Min.
- Komponente B: 3 Gew.-Tle. 1 M HCl in Wasser
12 Gew.-Tle. Emulgator
17 Gew.-Tle. Glycerin
10 Gew.-Tle. Paraffin (Vaseline)
55 Gew.-Tle. Füllstoffe (Quarzmehl)
3 Gew.-Tle. Aerosil (SiO₂)
werden in der angegebenen Reihenfolge gemischt. Die Mischzeit bei 25°C beträgt 30 Min. bei 50 U/Min.

Gleiche Gewichtsteile der Komponenten A und B werden intensiv vermischt und vernetzen zu einem elastischen Material innerhalb von 5 Minuten.

Die physikalischen Werte des Materials, gemessen nach ISO 4823, sind wie folgt:

| | |
|---|---|
| Verarbeitungszeit | 4 Min. |
| Bleibende Deformation | 1,1 %. |

Verträglichkeit mit Gips: keine Veränderungen

Die Dimensionsänderung, gemessen nach ADA 19 bei Trockenlagerung, beträgt:

| | |
|---|---|
| nach 24 Stunden | 0,2 % |

## Patentansprüche

1. Verwendung von Mischungen enthaltend
A) ein Ether-, Urethan-, Harnstoffgruppen und Alkoxysilylendgruppen enthaltendes Polyadditionsprodukt mit einer überwiegend linearen Molekülstruktur, ausschließlich aliphatisch oder cycloaliphatisch gebundenen Ether-, Urethan- und Harnstoffsegmenten und einem mittleren Molekulargewicht Mn von 800-20.000, gekennzeichnet durch
a) einen Gehalt von Polyethergruppen von 25-90 Gew.-%
b) einen Gehalt von Urethangruppen (-NH-CO-O-) von 0,5-10 Gew.-%
c) einen Gehalt von Harnstoffgruppen (-NH-CO-NH-) von 0,5-10 Gew.-% und
d) endständige Gruppen der Formel
-NR-(CH₂)ₙ-SiR₁R₂R₃,
worin n die Zahlen 1-6 darstellt,
R Wasserstoff oder -(CH₂)ₙ-SiR₁R₂R₃ bedeutet,
R₁, R₂, R₃ unabhängig voneinander C₁-C₄-Alkoxy bedeuten, wobei der Gehalt der endständigen Alkoxysilylgruppen -SiR₁R₂R₃ 1-25 Gew.-% beträgt und
B) eine Abmischung enthaltend Wasser und organische und/oder anorganische Säuren in Gewichtsmengenverhältnissen von 1:0,01 bis 1:40 als Abform- oder Dubliermassen im Dentalbereich.

2. Verwendung von Mischungen nach Anspruch 1, dadurch gekennzeichnet, daß die Komponente A)
a) einen Gehalt von Polyethergruppen von 50-80 Gew.-%,
b) einen Gehalt von Urethangruppen (-NH-CO-O-) von 1-8 Gew.-%,
c) einen Gehalt von Harnstoffgruppen (-NH-CO-NH-) von 1-8 Gew.-% aufweist und der Gehalt der endständigen Alkoxysilylgruppen -SiR₁R₂R₃ der endständigen Gruppen (d) 2-10 Gew.-% beträgt.

3. Verwendung von Mischungen nach Ansprüchen 1 und 2, dadurch gekennzeichnet, daß die Komponente B Wasser und organische und/oder anorganische Säuren in Gewichtsverhältnissen von 1:0,05 bis 1:1 enthält.

## Claims

1. Use of mixtures containing
A) a polyaddition product which contains ether, urethane and urea groups and alkoxysilyl end groups and has a predominantly linear molecular structure, exclusively aliphatically or cycloaliphatically bonded ether, urethane and urea segments and an average molecular weight Mn of 800 - 20,000, characterised in that they contain
a) 25 to 90% by weight of polyether groups,
b) 0.5 to 10% by weight of urethane groups (-NH-CO-O-),
c) 0.5 to 10% by weight of urea groups (-NH-CO-NH-) and
d) terminal groups of the formula
-NR-(CH₂)ₙ-SiR₁R₂R₃,
wherein n represents the numbers 1 to 6,
R denotes hydrogen or -(CH₂)ₙ-SiR₁R₂R₃,
R₁, R₂, R₃ independently of one another denote C₁-C₄-alkoxy, the content of the terminal alkoxysilyl groups -SiR₁R₂R₃ amounting to 1-25% by weight and
B) a mixture containing water and organic and/or inorganic acids in weight ratios of 1:0.01 to 1:40 as impression or duplicating compositions in the dental field.

2. Use of mixtures according to Claim 1, characterised in that component A) contains
a) 50 to 80% by weight of polyether groups,
b) 1 to 8% by weight of urethane groups (-NH-CO-O-),
c) 1 to 8% by weight of urea groups (-NH-CO-NH-) and the content of the terminal alkoxysilyl groups -SiR₁R₂R₃ of the terminal groups (d) amounts to 2 to 10% by weight.

3. Use of mixtures according to Claims 1 and 2, characterised in that component B contains water and organic and/or inorganic acids in weight ratios of 1:0.05 to 1:1.

## Revendications

1. Utilisation de mélanges contenant
A) un produit de polyaddition contenant des groupes éther, uréthanne, urée et des groupes terminaux alkoxysilyle, ayant une structure moléculaire principalement linéaire, des segments éther, uréthanne et urée en liaison exclusivement aliphatique ou cycloaliphatique et un poids moléculaire moyen Mn de 800 à 20 000, caractérisée par
a) une teneur en groupes polyéther de 25 à 90 % en poids
b) une teneur en groupes uréthanne (-NH-CO-O-) de 0,5 à 10 % en poids
c) une teneur en groupes urée (-NH-CO-NH-) de 0,5 à 10 % en poids et
d) des groupes terminaux de formule
-NR-(CH₂)ₙ-SiR₁R₂R₃,
dans laquelle n représente les nombres de 1 à 6,
R est de l'hydrogène ou un groupe -(CH₂)ₙ-SiR₁R₂R₃,
R₁, R₂, R₃ représentent indépendamment les uns des autres des groupes alkoxy en C₁ à C₄, la teneur en groupes alkoxysilyle -SiR₁R₂R₃ terminaux s'élevant à 1-25 % en poids et
B) un mélange contenant de l'eau et des acides organiques et/ou inorganiques dans des rapports de quantités en poids de 1:0,01 à 1:40 comme compositions pour empreintes ou pour répliques dans l'art dentaire.

2. Utilisation de mélanges suivant la revendication 1, caractérisée en ce que le composant A) présente
a) une teneur en groupes polyéther de 50 à 80 % en poids,
b) une teneur en groupes uréthanne (-NH-CO-O-) de 1 à 8 % en poids,
c) une teneur en groupes urée (-NH-CO-NH-) de 1 à 8 % en poids, et la teneur en groupes alkoxysilyle -SiR₁R₂R₃ des groupes terminaux (d) s'élève à 2-10 % en poids.

3. Utilisation de mélanges suivant les revendications 1 et 2, caractérisée en ce que le composant B contient de l'eau et des acides organiques et/ou inorganiques dans des rapports en poids de 1:0,05 à 1:1.
